# EUROPÄISCHE PATENTSCHRIFT

(11) **EP 3 630 220 B1**
(45) Veröffentlichungstag und Bekanntmachung des Hinweises auf die Patenterteilung: **22.03.2023**
(21) Anmeldenummer: 18728358.5
(22) Anmeldetag: 30.05.2018
(51) Int. Cl.: A61M 1/28, A61M 1/16, G01G 21/22, G01G 23/37, G01G 19/387

(54) **VORRICHTUNG UND VERFAHREN ZUR HERSTELLUNG EINER PERITONEALDIALYSELÖSUNG**
DEVICE AND METHOD FOR PRODUCING A PERITONEAL DIALYSIS SOLUTION
DISPOSITIF ET PROCÉDÉ DE FABRICATION D'UNE SOLUTION DE DIALYSE PÉRITONÉALE

(30) Priorität: 30.05.2017 DE 102017111803
(43) Veröffentlichungstag der Anmeldung: 08.04.2020
(73) Patentinhaber: Fresenius Medical Care Deutschland GmbH, 61352 Bad Homburg (DE)
(72) Erfinder: WABEL, Peter, 64287 Darmstadt (DE)
(74) Vertreter: Herrmann, Uwe
(86) Internationale Anmeldenummer: PCT/EP2018/064134
(87) Internationale Veröffentlichungsnummer: WO 2018/219981

(56) Entgegenhaltungen:
- EP-A1- 0 619 135
- WO-A1-95/20985
- WO-A1-2016/087043
- US-A- 4 585 436

## Beschreibung

Die Erfindung betrifft eine Vorrichtung für die Durchführung einer Point-of-Care Peritonealdialysebehandlung und ein Verfahren für die Herstellung einer Peritonealdialyselösung am Anwendungsort.

Die Peritonealdialyse (PD) ist auch unter dem Begriff Bauchfelldialyse bekannt. Es gibt unterschiedliche PD-Verfahren, darunter die mit Peritonealdialysegeräten durchgeführten Verfahren der automatisierte Peritonealdialyse (APD). In diesen Verfahren führt das Gerät dem Patienten in einer Einlaufphase über einen Katheter eine Dialyselösung in die Bauchhöhle.

Der Zulauf von Dialyselösung zum Patienten und/oder der Ablauf gebrauchter Dialyselösung vom Patienten kann mittels einer oder mehrerer Pumpen und/oder gravimetrisch erfolgen, d.h. schwerkraftbedingt. Von der Erfindung sind grundsätzlich beide Varianten sowie auch eine Kombination von diesen umfasst.

Die Dialyselösung wird dann während einer Halteperiode in der Bauchhöhle belassen. Dabei können kleinmolekulare Substanzen aus dem Blut über die Kapillargefäße des Bauchfells in die Dialyselösung übertreten, da ein Konzentrationsgefälle herrscht. Ferner kann dem Körper auf diesem Wege auch Wasser entzogen werden, sofern die Dialyselösung einen höheren Gehalt an osmotisch aktiven Substanzen aufweist als das Blut. Nach Ende der Halteperiode entfernt das Gerät die mit ausgeschiedenen Substanzen angereicherte und mithin verbrauchte Dialyselösung in einer Ablaufphase über den Katheter wieder aus der Bauchhöhle.

Bei einer sogenannten Point-of-Care Peritonealdialyse (PoC-PD) wird die Dialyselösung am Anwendungsort hergestellt, beispielsweise direkt beim Patienten zuhause. Hierzu kann beispielsweise die Verdünnung eines Konzentrats oder eine anderweitige Vermischung unterschiedlicher Ausgangskomponenten vorgesehen sein. Die vor Ort hergestellte Lösung wird dem Patienten zur Verfügung gestellt oder vom Peritonealdialysegerät direkt appliziert.

Die Druckschrift EP0619135A1 offenbart ein System zur Herstellung einer Lösung aus mehreren Komponenten mittels einer Pumpe, mittels welcher sich beliebige Mischungsverhältnisse durch eine entsprechende Steuerung der Pumpe einstellen lassen.

Aufgabe der Erfindung ist es, eine Lösung bereitzustellen, mit der eine besonders einfache und fehlerunanfällige Herstellung einer Peritonealdialyselösung am Anwendungsort und Durchführung einer Point-of-Care Peritonealdialyse ermöglicht wird.

Vor diesem Hintergrund betrifft die Erfindung eine Vorrichtung zur Herstellung einer Peritonealdialyselösung am Anwendungsort umfassend ein Disposable mit einem oder mehreren Lösungsbeuteln und damit verbundenen Füll- und Patientenleitungen, eine mit der Füllleitung verbundene Bereitstellungseinheit für Flüssigkeit, eine Steuereinheit und Aktoren zur Regelung eines Flusses von der Bereitstellungseinheit zum Disposable. Erfindungsgemäß ist vorgesehen, dass die Vorrichtung ferner eine Waage umfasst, an welcher der Lösungsbeutel des Disposables angeordnet ist, wobei die Steuereinheit ausgebildet ist, anhand der Aktoren eine Befüllung des Lösungsbeutels mit Flüssigkeit von der Bereitstellungseinheit zu veranlassen und anhand der Waage die Menge der in den Lösungsbeutel geflossenen Flüssigkeit zu bestimmen und dazu ausgebildet ist, den Befüllvorgang nach Erreichen einer vorge-gebenen Füllmenge zu stoppen und anhand der Waage zu verifizieren, ob das Gewicht des Beutels nach dem Stopp des Befüllvorgangs in einem Sollbereich liegt, und weiter dazu ausgebildet ist, die Grenzen des Sollbe-reichs durch Addition eines bestimmten Betrags zum ermittelten Ausgangs-gewicht des Beutels oder zu einem nach dem vorhergehenden Schritt er-mittelten Gewicht des Beutels festzulegen.. Die Waage kann eine Auflagefläche und insbesondere Auflageschale oder eine Aufhängvorrichtung für den Beutel aufweisen.

Der oder die Lösungsbeutel kann auf der Waage aufliegen, an dieser angehängt sein etc. Auch ist es denkbar, dass die Waage Einschübe aufweist, in denen ein oder mehrere Lösungsbeutel aufgenommen werden.

An dieser Stelle wird darauf hingewiesen, dass die Begriffe "ein" und "eine" nicht zwingend genau eines der fraglichen Elemente betreffen, wenngleich dies eine denkbare Ausführungsform ist, sondern auch deren Mehrzahl umfasst. Ebenso umfasst die Verwendung des Singulars eines Elementes auch dessen Mehrzahl und umgekehrt.

Des Weiteren wird darauf hingewiesen, dass der Begriff "Lösungsbeutel" allgemein und stellvertretend für jedes beliebige Behältnis steht, das die Lösung enthält oder in dem die Lösung aufgenommen werden kann. Es kann sich dabei um einen Container, Beutel im engeren Sinne oder um ein sonstiges Behältnis mit starren und/oder flexiblen Wandungen handeln.

In einer Ausführungsform ist vorgesehen, dass die Vorrichtung eine Pumpe umfasst und die Steuereinheit ausgebildet ist, eine aktive Förderung der Flüssigkeit an den Lösungsbeutel zu veranlassen. Es kann vorgesehen sein, dass es sich bei der Pumpe um eine bilanzierende Pumpe handelt, mit welcher das an den Lösungsbeutel geförderte Flüssigkeitsvolumen bestimmt werden kann.

In einer Ausführungsform ist vorgesehen, dass die Vorrichtung einen Flussregler umfasst und die Steuereinheit ausgebildet ist, anhand des Flussreglers einen gravimetrischen Fluss der Flüssigkeit in den Lösungsbeutel zu veranlassen. Bei dem Flussregler kann es sich beispielsweise um ein Ventil oder eine Klemme handeln.

In einer Ausführungsform ist vorgesehen, dass die Steuereinheit ausgebildet ist, die in den Lösungsbeutel gefüllte Flüssigkeitsmenge anhand einer bilanzierenden Pumpe zu steuern oder auf der Grundlage des Signals eines Flusssensors zu regeln und das Signal der Waage lediglich zur Überprüfung der in den Lösungsbeutel gefüllte Flüssigkeitsmenge heranzuziehen. Alternativ oder zusätzlich zu einer bilanzierenden Pumpe kann zur Bestimmung der in den Beutel fließenden Flüssigkeitsmenge auch ein Flusssensor vorgesehen sein.

In einer Ausführungsform ist vorgesehen, dass die Steuereinheit ausgebildet ist, die in den Lösungsbeutel gefüllte Flüssigkeitsmenge anhand des Signals der Waage zu regeln. Es kann also vorgesehen sein, dass das Signal der Waage von der Steuereinheit zur Regelung des Flusses an den Lösungsbeutel verwendet wird. In dieser Ausführungsform sind eine bilanzierende Pumpe und ein Flusssensor entbehrlich.

In einer Ausführungsform ist vorgesehen, dass der Lösungsbeutel mit einem Dialyseflüssigkeits-Konzentrat vorgefüllt ist, dass es sich bei der Bereitstellungseinheit um eine Wasseraufbereitungseinheit handelt, und dass es sich bei der in den Lösungsbeutel gefüllten Flüssigkeit um Wasser handelt. Beim Befüllen mit Wasser wird das Konzentrat zur Herstellung der Dialyselösung verdünnt. Bei dem Dialyseflüssigkeits-Konzentrat kann es sich um ein festes oder flüssiges Konzentrat handeln. Beispielsweise kann der Lösungsbeutel ein flüssiges Lösungskonzentrat enthalten, das vor Anwendung beispielsweise mit der 10- bis 20-fachen Menge an sterilem Wasser verdünnt werden muss, um eine gebrauchsfertige Dialyselösung zu erhalten. Es muss also einer definierten Menge an Konzentrat eine genau definierte Menge an sterilem Wasser zugeführt werden, sodass um eine gebrauchsfertige Dialyselösung mit den Bestandteilen in den richtigen Konzentrationen zu erhalten. Der Beutel kann Mittel zum Rückhalten des Konzentrats aufweisen, beispielsweise trennbare Schweißnähte oder dergleichen.

Der Lösungsbeutel kann ein oder mehrere Kompartimente aufweisen. Im Falle mehrerer Kompartimente können diese beispielsweise mit Schweißnähten voneinander getrennt sein. Das Volumen des Lösungsbeutels ist groß genug, um die für einen Behandlungszyklus erforderliche Menge an Dialyseflüssigkeit aufzunehmen.

Weiterhin kann vorgesehen sein, dass sich unter dem oder den Lösungsbeuteln (z.B. Diposablebeutel) eine Heizung befindet.

In einer Ausführungsform ist vorgesehen, dass die Bereitstellungseinheit ausgebildet ist, wenigstens zwei unterschiedliche fluide Lösungsbestandteile bereitzustellen, und die Steuereinheit ausgebildet ist, eine sequentielle Befüllung des Lösungsbeutel mit den unterschiedlichen Lösungsbestandteilen zu veranlassen. Bei den fluiden Lösungsbestandteilen kann es sich um reines Wasser sowie um konzentrierte Teillösungen und/oder verdünnte Teillösungen handeln. Geeignete Teillösungen umfassen beispielsweise eine Glukoselösung oder eine Elektrolytlösung. Beispielsweise kann der Lösungsbeutel in drei Schritten sequentiell mit Wasser, mit einer Glukoselösung und mit einer Elektrolytlösung befüllt werden. Es können auch mehrere Wiederholungen einer Sequenz an Schritten vorgesehen sein, also beispielsweise ein erster Schritt, in dem der Lösungsbeutel mit einer bestimmten Menge einer ersten Teillösung befüllt wird, gefolgt von einem zweiten Schritt, in dem der Lösungsbeutel mit einer bestimmten Menge einer zweiten Teillösung befüllt wird, gefolgt von einem dritten Schritt, in dem der Lösungsbeutel wiederum mit einer bestimmten Menge der ersten Teillösung befüllt wird, und so weiter. Der Lösungsbeutel kann in dieser Ausführungsform vor dem Befüllen leer sein, es kann aber alternativ auch hier eine Vorbefüllung mit einem Konzentrat vorgesehen sein.

In einer Ausführungsform ist vorgesehen, dass die Bereitstellungseinheit einen Umkehrosmosefilter umfasst, um steriles und reines Wasser herzustellen.

In einer Ausführungsform ist vorgesehen, dass die Steuereinheit ausgebildet ist, ein Signal auszugeben und/oder einen Start des Befüllvorgangs zu unterbinden oder den Befüllvorgang anzupassen, wenn das anhand der Waage ermittelte Ausgangsgewicht des Beutels einen Schwellenwert unter- oder überschreitet. Für das Gewicht des leeren oder mit Konzentrat befüllten Lösungsbeutels vor Beginn des Befüllvorgangs ist in der Steuereinheit ein Sollwert hinterlegt oder wird an diese übertragen oder befindet sich an dem Lösungsbeutel, z.B. in Form eines Barcodes und wird von der Steuereinheit ausgelesen. Anhand der Waage kann die Steuereinheit also das Gewicht des Lösungsbeutels vor Beginn des Befüllvorgangs bestimmen und kann somit verifizieren, ob ein Beutel mit dem korrekten Gewicht richtig eingelegt wurde oder ob der Beutel Flüssigkeit durch eine Leckage oder falsche Lagerung verloren hat. Der Sollbereich kann einem in der Steuereinheit hinterlegten Sollwert inklusive einem Toleranzbereich von beispielsweise 5%, 2% oder 1 % in jede Richtung entsprechen. Der Sollbereich kann auch als Absolutwert berechnet werden bzw. vorliegen.

Der Sollwert kann sich aus der Verschreibung ergeben. Ferner kann vorgesehen sein, dass die Vorrichtung eine Leseeinrichtung umfasst und die Steuereinheit ausgebildet ist, den Sollwert vor einzulesen. In diesem Zusammenhang kann vorgesehen sein, dass das Beutelgewicht bereits in der Produktion bestimmt wird und das gemessene Gewicht am Beutel festgehalten wird, beispielsweise anhand eines Barcodes oder eines RFID-Chips. Vor Beginn des Befüllvorgangs kann dann das dokumentierte Gewicht mit dem tatsächlich an der Vorrichtung gemessenen Gewicht verglichen werden. Abweichungen können auf eine fehlerhafte Lagerung oder auf eine Beschädigung hinweisen.

In einer Ausführungsform ist vorgesehen, dass die Steuereinheit ausgebildet ist, den Befüllvorgang nach Benutzereingabe zu starten.

Erfindungsgemäß ist vorgesehen, dass die Steuereinheit ausgebildet ist, den Befüllvorgang nach Erreichen einer vorgegebenen Füllmenge zu stoppen und anhand der Waage zu verifizieren, ob das Gewicht des Beutels nach dem Stopp des Befüllvorgangs in einem Sollbereich liegt. Das Stoppen des Befüllvorgangs erfolgt beispielsweise durch ein Stoppen der Pumpe oder durch den Flussregler, beispielsweise durch das Schließen einer Klemme oder durch einen Hinweis an den Nutzer, der dann den Fluss stoppt. Bei dem Stopp des Befüllvorgangs kann es sich um einen finalen Stopp am Ende des gesamten Befüllvorgangs oder um einen Zwischenstopp nach einem Schritt eines mehrstufigen Befüllvorgangs handeln, in dem der Lösungsbeutel in sequentiellen Schritten mit unterschiedlichen Lösungsbestandteilen befüllt wird. Anhand der Waage kann die Steuereinheit also das Gewicht des Lösungsbeutels nach dem Befüllvorgang oder jeweiligen Schritt des Befüllvorgangs bestimmen und somit verifizieren, ob im Rahmen des Befüllvorgangs oder des vorhergehenden Schrittes eine korrekte Flüssigkeitsmenge in den Lösungsbeutel geflossen ist. Der Sollbereich kann einem in der Steuereinheit hinterlegten Sollwert inklusive einem Toleranzbereich von beispielsweise 5%, 2% oder 1% in jede Richtung entsprechen. Auch hierbei sind auch Absolutwerte als Toleranzbereiche denkbar und von der Erfindung mit umfasst.

Der Sollwert ist vorzugsweise in der Steuereinheit hinterlegt und kann sich aus der Verschreibung ergeben.

Anhand der Waage kann die Steuereinheit also das Gewicht des Lösungsbeutels vor dem Befüllvorgang, nach jedem etwaigen Zwischenstopp des Befüllvorgangs und nach dem Ende des Befüllvorgangs bestimmen und so verifizieren, ob eine korrekte Flüssigkeitsmenge in den Lösungsbeutel geflossen ist.

Erfindungsgemäß ist vorgesehen, dass die Steuereinheit ausgebildet ist, die Grenzen des Sollbereichs durch Addition eines bestimmten Betrags zum ermittelten Ausgangsgewicht des Beutels oder zu einem nach dem vorhergehenden Schritt ermittelten Gewicht des Beutels festzulegen. Der Sollwert und die Grenzen des Sollbereichs sind somit nicht als absolute Zahlen fest vorgegeben, sondern nur als Differenzbetrag zum Gewicht des leeren Beutels oder teilbefüllten Beutels. Würde man absolute Zahlen festlegen, so bestünde das Risiko, dass dann, wenn beispielsweise das Gewicht des leeren Beutels nicht exakt dem theoretischen Sollgewicht entsprechen sondern innerhalb des Toleranzbereichs davon abweichen würde, die Gewichtsverifikation des nächsten Schrittes um den Betrag der Abweichung verfälscht wäre. Dieses Problem wird durch die Referenzierung auf das tatsächlich ermittelte Gewicht vermieden.

Es ist auch möglich, dass bei einem schrittweisen Füllen evtl. ein zu großes/zu geringes Füllvolumen in einem der nachfolgenden Schritte korrigiert werden kann bzw. muss.

In einer Ausführungsform ist vorgesehen, dass die Steuereinheit ausgebildet ist, nach dem Ende des Befüllvorgangs ein Signal auszugeben, um eine Bewegung des befüllten Lösungsbeutels zu fordern, und anhand der Waage zu verifizieren, ob der Lösungsbeutel tatsächlich bewegt wurde. So kann der Anwender veranlasst werden, den Lösungsbeutel nach dessen vollständiger Befüllung zur homogenen Durchmischung der Lösungsbestandteile zu bewegen. Beispielsweise kann der Benutzer den Beutel von der Waage, insbesondere von der Auflagefläche oder Aufhängevorrichtung abnehmen und wieder auf die Waage legen, oder er kann ihn auf der Waage bewegen. Die Bewegung kann anhand der Waage insofern verifiziert werden, als eine Störung und anschließende Wiedereinstellung des Endgewichts des Beutels erkannt werden kann.

Zum Zwecke der Durchmischung kann auch eine Schweißnaht geöffnet werden, die vor ihrer Öffnung verschiedene Lösungsteile voneinander trennt.

In einer Ausführungsform ist vorgesehen, dass die Vorrichtung einen Aktor aufweist, der ausgebildet ist, um den eingelegten Lösungsbeutel bewegen zu können, und dass die Steuereinheit ausgebildet ist, den Lösungsbeutel nach dem Ende des Befüllvorgangs zu bewegen. Beispielsweise kann ein Motor vorgesehen sein, der eine Vibration der Auflagefläche oder Aufhängvorrichtung der Waage verursacht. So kann eine homogene Durchmischung erreicht werden, ohne dass der Benutzer den Lösungsbeutel abnehmen muss.

In einer Ausführungsform ist vorgesehen, dass die Steuereinheit ausgebildet ist, ein Signal auszugeben und/oder die Pumpe der Bereitstellungseinheit zu stoppen und/oder die Füllklemme zu schließen, wenn während des Befüllvorgangs anhand der Waage eine Stagnation der Gewichtszunahme oder eine Gewichtsabnahme des Lösungsbeutels erkannt wird. Durch ein Schließen der Füllklemme kann ein unerwünschtes Rückströmen von Dialyselösung in die Wasseraufbereitungseinheit verhindert werden.

In einer Ausführungsform ist vorgesehen, dass die Vorrichtung eine Heizung aufweist und die Steuereinheit ausgebildet ist, den Beutel während des Befüllvorgangs oder nach dem Befüllvorgang zu erwärmen.

In einer Ausführungsform ist vorgesehen, dass in der Füllleitung ein Rückschlagventil angeordnet ist, das einen Fluss vom Beutel zur Wasseraufbereitungseinheit unterbindet. Das Rückschlagventil ist in dieser Ausführungsform also im Disposable angeordnet. So kann im Falle eines Fehlers während des Befüllvorgangs ein Rückströmen von Dialyselösung in die Befülleinheit verhindert werden oder eine maschinenseitige Verhinderungsmaßnahme unterstützt werden.

Die Füllleitung kann sich auf derselben oder der anderen Seite des Lösungsbeutels befinden wie die Patientenleitung. Bevorzugt ist eine Ausgestaltung, bei der sich die Leitungen auf gegenüberliegenden Seiten befinden.

In einer Ausführungsform ist vorgesehen, dass es sich bei der erfindungsgemäßen Vorrichtung um ein Dialysegerät handelt, vorzugsweise um ein Gerät zur Durchführung einer automatisierten Peritonealdialyse im Rahmen einer Point-of-Care Dialysebehandlung. Hierbei kann vorgesehen sein, dass die Steuereinheit ausgebildet ist, nach Ende des Befüllvorgangs automatisch eine Dialysebehandlung zu starten. Alternativ kann vorgesehen sein, die Steuereinheit ausgebildet ist, nach einem erfolgreichen Befüllen einen Schalter oder ein Feld auf einem Touchscreen des Benutzerinterfaces freizuschalten, anhand dessen eine Dialysebehandlung manuell gestartet werden kann. Denkbar ist auch nur ein Hinweissignal, dass der Anwender die Behandlung startet.

Das Freischalten kann auch das Freischalten der Patientenklemme sein.

In einer Ausführungsform ist vorgesehen, dass die Vorrichtung eine Füllklemme und eine Patientenklemmen aufweist, wobei die Füllleitung des Disposables in die Füllklemme und die Patientenleitung in die Patientenklemme eingelegt ist, und wobei die Steuereinheit ausgebildet ist, die Befüllung des Lösungsbeutels bei offener Füllklemme und geschlossener Patientenklemme vorzunehmen.

In einer Ausführungsform ist vorgesehen, dass die Vorrichtung ein weiteres Disposable oder das Disposable eine zusätzliche Kavität für aus dem Patienten abfließendes Dialysat aufweist, das oder die auf derselben Waage angeordnet ist wie der Lösungsbeutel, wobei die Steuereinheit ausgebildet ist, die Menge des im Rahmen einer Dialysebehandlung von dem Patienten abfließenden Dialysats gravimetrisch zu bestimmen. So kann auch die Menge des abfließenden Dialysats gravimetrisch überwacht werden.

Das abfließende Dialysat kann auch mittels einer Pumpe in den Beutel gepumpt werden.

In einer Ausführungsform ist vorgesehen, dass die Vorrichtung ein graphisches Benutzerinterface mit vorzugsweise einem Touchscreen aufweist und die Steuereinheit ausgebildet ist, am Benutzerinterface den Fortschritt des Befüllvorgangs zu visualisieren und/oder Signale auszugeben. Beispielsweise kann vorgesehen sein, dass bei positivem Abschluss der Bestimmung des Anfangsgewichts des Beutels und/oder bei positivem Abschluss des Befüllvorgangs auf einem Touchscreen des Benutzerinterfaces ein Feld erscheint, das zum Start der Beutelbefüllung bzw. zum Start der Dialysebehandlung betätigt werden kann. Sofern die jeweilige Prüfung negativ ausfällt, so wird ein Start der Beutelbefüllung bzw. Dialysebehandlung nicht freigegeben und am Bildschirm erscheint vielmehr ein Warnhinweis und eine geeignete Aufforderung zur Handlung.

Vor dem eingangs genannten Hintergrund betrifft die Erfindung ferner ein Verfahren zur Herstellung einer Peritonealdialyselösung am Anwendungsort unter Verwendung einer erfindungsgemäßen Vorrichtung, wobei anhand der Aktoren eine Befüllung des Lösungsbeutels mit Flüssigkeit von der Bereitstellungseinheit veranlasst wird und wobei anhand der Waage die Menge der in den Lösungsbeutel geflossenen Flüssigkeit bestimmt wird. Vorteilhafte Ausgestaltungen des Verfahrens ergeben sich aus der obigen Beschreibung der Ausbildung der Steuereinheit in der erfindungsgemäßen Vorrichtung. Es ist anzumerken, dass der Befüllvorgang ohne Beisein des Patienten durchgeführt werden kann. Ein Beisein des Patienten ist erst während der dem Befüllvorgang nachgelagerten Dialysebehandlung erforderlich.

Das Befüllen kann automatisch oder durch das Pflegepersonal erfolgen.

Weitere Einzelheiten und Vorteile der Erfindung ergeben sich aus dem nachfolgend anhand der Figuren erklärten Ausführungsbeispiel. In den Figuren zeigen:
- Figur 1:: eine schematische Darstellung von Teilen einer erfindungsgemäßen Vorrichtung in einer ersten Ausführungsform;
- Figur 2:: eine schematische Darstellung eines Disposables der Vorrichtung;
- Figur 3:: ein Verlaufsdiagramm des an der Waage der Vorrichtung gemessenen Beutelgewichts;
- Figur 4:: ein weiteres Verlaufsdiagramm zur Veranschaulichung bestimmter Störungen;
- Figur 5:: weitere Verlaufsdiagramme zur Veranschaulichung bestimmter Teilaspekte;
- Figur 6:: Ausschnitte aus den Fluidschemata einer zweiten und dritten Ausführungsform einer erfindungsgemäßen Vorrichtung;
- Figur 7:: ein Verlaufsdiagramm des an der Waage der Vorrichtungen gemäß zweiter und dritter Ausführungsform;
- Figur 8:: weitere Verlaufsdiagramme zur Veranschaulichung bestimmter Teilaspekte;
- Figur 9:: zusätzliche Verlaufsdiagramme zur Veranschaulichung wiederum anderer Teilaspekte; und
- Figur 10:: eine Teilansicht eines Geräts zur Veranschaulichung eines weiteren Teilaspekts.

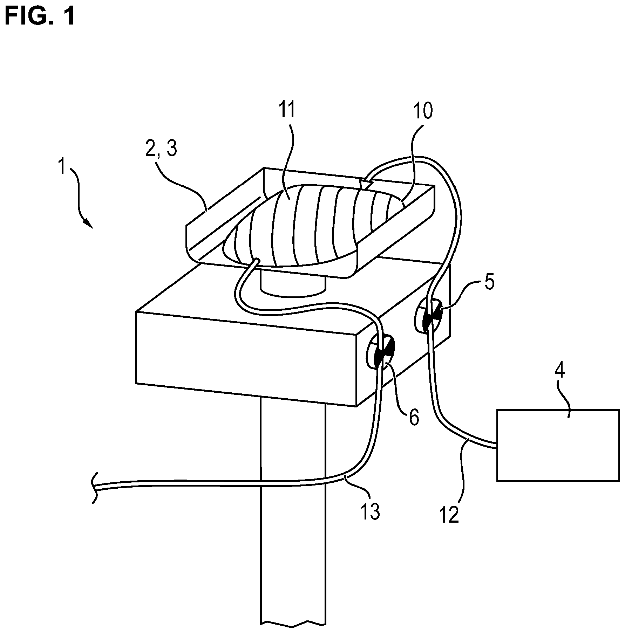

Figur 1 zeigt schematisch eine erfindungsgemäße Vorrichtung 1, die als gravimetrisches Peritonealdialysegerät zur Durchführung einer automatisierten Point-of-Care Peritonealdialyse ausgebildet ist.

Das Gerät 1 umfasst eine Waage 2 mit einer Waagschale 3, eine Flüssigkeitsbereitstellungseinheit 4 mit einem Umkehrosmosefilter zur Herstellung sterilen Wassers und eine in der Figur nicht dargestellte Steuereinheit. Das Gerät 1 weist ferner eine Füllklemme 5 und eine Patientenklemme 6 auf.

Es können auch mehrere Füllklemmen zum Füllen von mehreren Beuteln verschiedener Konzentration vorhanden sein.

In das Gerät ist wenigstens ein Disposable 10 eingelegt, das einen oder mehrere Lösungsbeutel 11 sowie eine Füllleitung 12 und eine Patientenleitung 13 umfasst. Der eingelegte Lösungsbeutel 11 befindet sich in der Waagschale 3, die Füllleitung 12 verläuft durch die Füllklemme 5 und die Patientenleitung 13 verläuft durch die Patientenklemme 6. Die Füllleitung 12 ist mit der Bereitstellungseinheit 4 verbunden. Die Patientenleitung 13 wird mit einem Katheter eines Patienten verbunden.

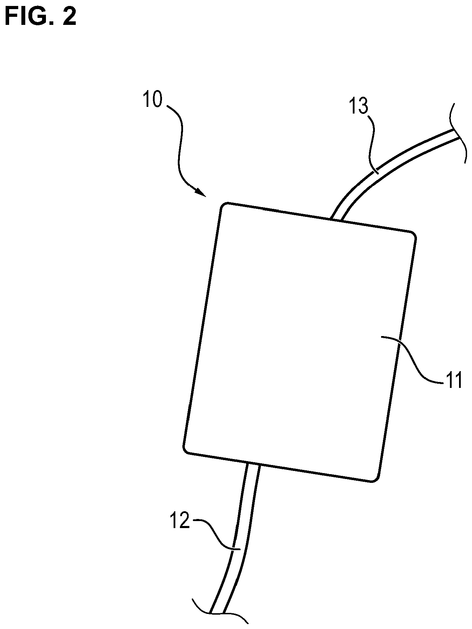

Das Disposable ist in Figur 2 näher dargestellt. Im Beutel 11 ist ein flüssiges Dialyseflüssigkeits-Konzentrat aufgenommen, das mit einer definierten Menge an sterilem Wasser verdünnt werden muss, um eine fertige Peritonealdialyselösung zu erhalten. Damit das Konzentrat vor dem Einlegen des Disposables 10 in das Gerät nicht ausfließen kann, sind am Beutel 11 in der Figur nicht dargestellte Schweißnähte vorgesehen, die nach dem Einlegen vom Benutzer geöffnet werden können.

Wie ausgeführt, kann auch eine Schweißnaht geöffnet werden, ein Deckel abgeschraubt, ein Konus gebrochen werden. Alle denkbaren Methoden zum Vermischen sind von der Erfindung umfasst.

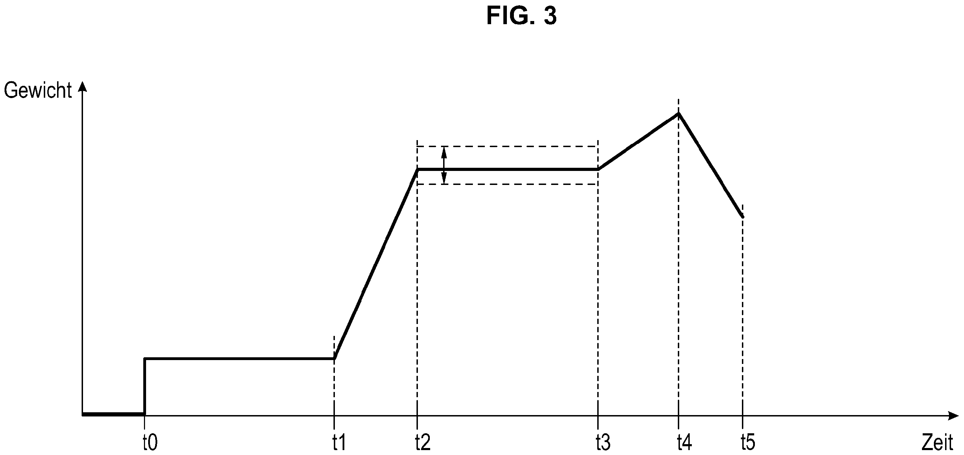

Nach Einlegen des Disposables 10 und Aktivierung der Vorrichtung 1 fragt die Steuereinheit zunächst das anhand der Waage 2 bestimmte Gewicht des mit Konzentrat gefüllten Beutels 11 vor Verdünnung mit Wasser ab. Das Sollgewicht dieses Beutels ist bekannt und in der Steuereinheit hinterlegt oder an diese übertragen. Es wird überprüft, ob das gemessene Gewicht mit dem Sollgewicht übereinstimmt bzw. innerhalb eines Toleranzbereichs um das Sollgewicht liegt. Sofern diese Prüfung positiv ausfällt, erscheint auf einem Touchscreen des in der Figur nicht dargestellten Benutzerinterfaces des Geräts 1 ein Feld, das zum Start der Beutelbefüllung betätigt werden kann. Es kann auch ein optischer und/oder akustischer Hinweis gegeben werden. Sofern diese Prüfung negativ ausfällt, sei es durch ein falsches oder defektes Disposable oder ein fehlerhaftes Einlegen, so wird ein Start der Beutelbefüllung nicht freigegeben und am Bildschirm erscheint vielmehr ein Warnhinweis und eine Aufforderung, das Disposable zu überprüfen oder ein Warnsignal ist zu hören. Dieser Schritt ist im zeitlichen Verlauf der Figur 3 als erste Stufe zwischen den Zeitpunkten t0 (Einlegen des Beutels) und t1 (Start des Befüllvorgangs) dargestellt.

Zu dem in der Figur 3 dargestellten Zeitpunkt t1 startet der Benutzer die Beutelbefüllung am eingelegten Disposable 10 durch Betätigung des freigeschalteten Aktivierungsfeldes am Benutzerinterface des Gerätes 1 oder durch Betätigen eines Schalters oder manuelles Öffnen einer Klemme. Die Steuereinheit schließt daraufhin die bisher offene Patientenklemme 6 und aktiviert bei noch offener Füllklemme 5 die Bereitstellungseinheit 4. So wird ein gravimetrischer oder durch eine Pumpe bewirkter Befüllvorgang gestartet, der zwischen den in der Figur 3 dargestellten Zeitpunkten t1 und t2 liegt. Während dieses Befüllvorgangs (Zeitspanne von t1 bis t2), der sich beispielsweise über einen Zeitraum von etwa drei Stunden erstrecken kann, gelangt durch die Füllleitung 12 langsam steriles Wasser in den Beutel 11. Dabei wird das im Beutel 11 befindliche Konzentrat fortlaufend verdünnt und das Beutelgewicht steigt, wie dies in Figur 3 zu erkennen ist, linear an. Ab t2 wird eine in der Figur nicht dargestellte Heizeinheit des Gerätes 1 aktiviert, um den Beutel 11 und den Beutelinhalt zu temperieren. Das Vorheizen sowie die Prüfung, ob das Beutelgewicht korrekt ist, erfolgt zwischen t2 und t3. Der mit der Waage 2 gemessene Anstieg des Beutelgewichts während des Befüllvorgangs wird von der Steuereinheit fortlaufend überwacht.

Ab t3 erfolgt der initiale Drain des Patienten und ab t4 (bis t5) das Befüllen des Patienten mit frischer Dialyselösung.

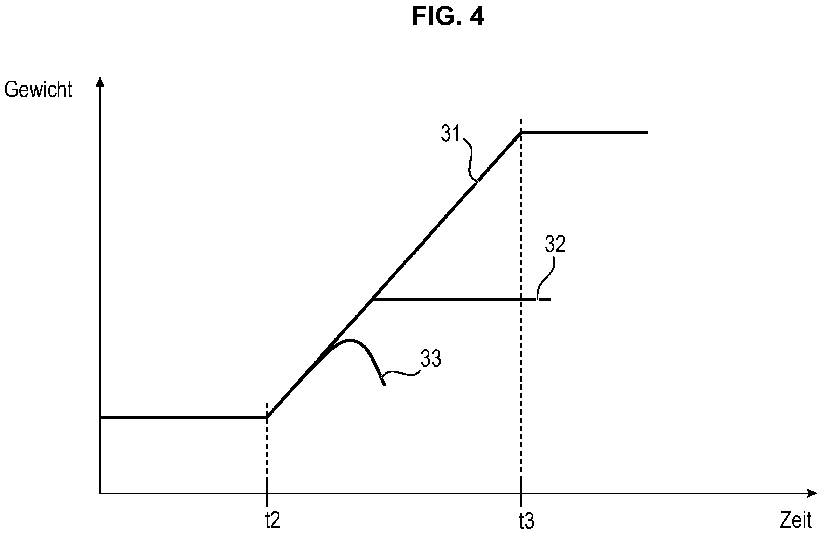

Im Verlaufsdiagramm gemäß Figur 4 sind zwei mögliche Fehler dargestellt, die durch fortlaufende Überwachung des Beutelgewichts während des Befüllvorgangs erkannt werden können. Eine erste Linie 31 zeigt den auch in Figur 3 erkennbaren Normalverlauf. Eine zweite Linie 32 zeigt eine Stagnation der Gewichtszunahme des Beutels 11, was beispielsweise durch ein Knicken oder ein Durchhängen der Füllleitung 12 bedingt sein kann. Eine dritte Linie 33 zeigt eine Gewichtsabnahme des Beutels 11, was beispielsweise durch eine unerwünschte Rückströmung oder ein Leck des Beutels 11 oder durch eine fehlerhaft geöffnete Patientenklemme bedingt sein kann. Wird eine Fehlfunktion wie im Falle der zweiten oder dritten Linie erkannt, wird die Füllklemme 5 sofort geschlossen und eine Fehlermeldung am Benutzerinterface des Geräts 1 ausgegeben.

Zum Zeitpunkt t2 der Figur 3 wird die Bereitstellungseinheit 4 abgeschaltet und die Füllklemme 5 geschlossen, da ein nicht näher dargestellter Flusssensor und/oder die Waage in der Bereitstellungseinheit 4 ein Signal ausgibt, dass der Beutel 11 theoretisch vollständig befüllt sein müsste. Die Steuereinheit startet nun einen Überprüfungsvorgang und misst anhand der Waage 2, ob der befüllte Beutel 11 ein bekanntes und in der Steuereinheit hinterlegtes Endgewicht erreicht hat. Es wird überprüft, ob das gemessene Gewicht mit dem hinterlegten Endgewicht übereinstimmt bzw. innerhalb eines Toleranzbereichs um das hinterlegte Endgewicht liegt. Sofern diese Prüfung positiv ausfällt, startet die Steuereinheit oder direkt die Waage des Geräts 1 zum vorgegebenen Zeitpunkt t3 automatisch eine Dialysebehandlung durch Öffnen einer Drainklemme (nicht dargestellt).

Sofern diese Prüfung negativ ausfällt, wird der Start der Dialysebehandlung nicht freigegeben und es können unterschiedliche Maßnahmen wie eine Nachbesserung oder eine Warnung an den Benutzer ergriffen werden.

Die Dialysebehandlung beginnt bei t3 mit einem initialen Abfluss von verbrauchtem Dialysat aus dem Patienten. Während dieses initialen Abflusses steigt das von der Waage 2 gemessene Gewicht an, da ein Abschnitt eines in der Figur nicht dargestellten weiteren Disposables für vom Patienten kommendes, verbrauchtes Dialysat ebenfalls über die Waage läuft. So kann anhand der erfindungsgemäßen Vorrichtung auch der Abfluss gravimetrisch auf einen reibungslosen Auslauf überwacht werden. Nach Ende des initialen Abflusses zum Zeitpunkt t4 wird die Patientenklemme 6 geöffnet und die Dialyseflüssigkeit fließt, bei geschlossener Füllklemme 5, gravimetrisch in die Bauchhöhle des Patienten, bis t5 erreicht ist.

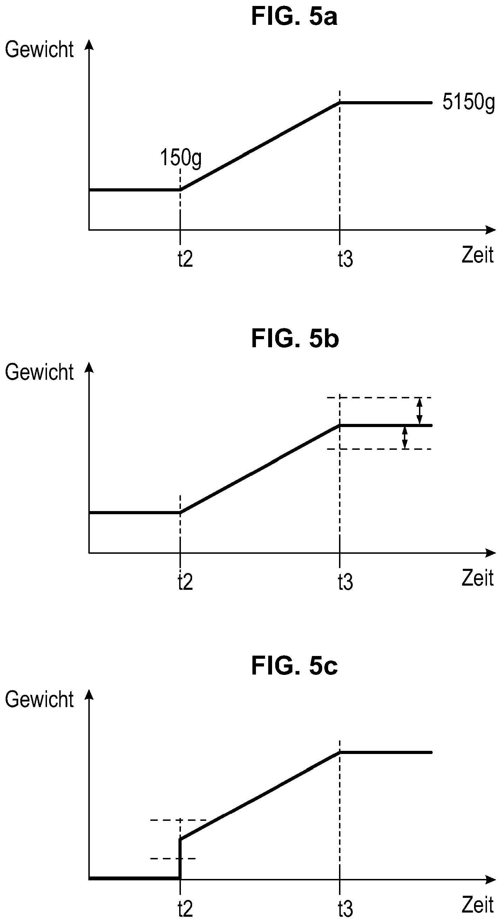

Figuren 5a bis 5c zeigen weitere Verlaufsdiagramme zur Veranschaulichung bestimmter Aspekte der Erfindung.

Figur 5a illustriert den Gewichtsverlauf des Beutels bei linearer Befüllung mit Wasser. Die Waage 2 misst das Gewicht des Beutels vor und nach der Befüllung (hier: 150 g bzw. 5150 g). Auf dieser Grundlage kann man verifizieren, dass die korrekte Menge an Wasser in den Flüssigkeitsbeutel 11 zugeführt wurde.

Figur 5b illustriert, dass ein Sollbereich (hier: bekannter Sollwert +/- 1% oder auch Absolutwerte) vorgesehen ist, innerhalb dessen das Gewicht des befüllten Beutels liegen soll. Der Sollbereich ist auf der Basis der zulässigen Toleranz der fertigen Lösung definiert. Sollte das Gewicht des gefüllten Beutels 11 außerhalb des Sollbereichs liegen, wird er nicht für die Therapie freigegeben.

Auch für das Gewicht des nur das Konzentrat enthaltenden, noch nicht mit Wasser gefüllten Beutels gibt es einen Sollbereich, wie dies in Figur 5c illustriert ist. Beim Einlegen des Disposables 10 bzw. beim Legen des Beutels 11 in die Waagschale 3 und mithin vor Beginn des Verdünnungsvorgangs wird das Gewicht des Beutels von der Steuereinheit abgefragt und auf Übereinstimmung mit dem Sollbereich überprüft. Sollte das Gewicht des leeren Beutels 11 außerhalb des Sollbereichs liegen, wird er nicht für den Befüllvorgang freigegeben oder es wird entsprechend mit mehr oder weniger Flüssigkeit verdünnt.

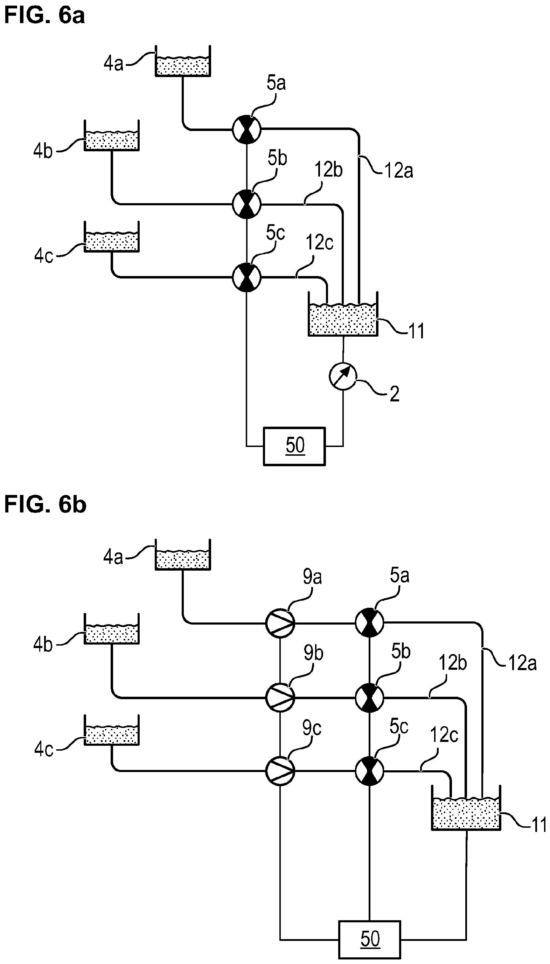

Die Figuren 6a und 6b zeigen Ausschnitte aus den Fluidschemata einer zweiten und dritten Ausführungsform eines Dialysegeräts der Erfindung. Der Grundaufbau des Dialysegeräts 1 ist dem Grundaufbau des in Figuren 1 bis 5 beschriebenen Geräts 1 ähnlich. Abweichend gestaltet sind lediglich die Mittel zur Beutelbefüllung. In beiden diesen Ausführungsformen umfasst die Bereitstellungseinheit mehrere Reservoirs 4a, 4b und 4c für unterschiedliche vorverdünnte Lösungsbestandteile. Ferner sind mehrere Füllleitungen 12a, 12b und 12c am Disposable 10 vorgesehen, die mit den einzelnen Reservoirs 4a, 4b und 4c in Verbindung stehen. Entsprechend umfasst das Gerät 1 auch mehrere Klemmen 5a, 5b und 5c für die unterschiedlichen Füllleitungen 4a, 4b und 4c. Der Beutel 1 ist initial nicht mit einem Konzentrat befüllt, sondern leer. Die Steuereinheit ist mit dem Bezugszeichen 50 gekennzeichnet und steht, unter anderem, mit den Klemmen 5a-5c und den Pumpen 9a-9c bzw. der Waage 2 in Verbindung.

In der zweiten Ausführungsform gemäß Figur 6a ist die Steuereinheit 50 ausgebildet, die in den Lösungsbeutel 11 gefüllte Flüssigkeitsmenge, also das Öffnen und Schließen der Klemmen 5a-5c anhand des Signals der Waage 2 zu regeln. Es wird alleine das Signal der Waage 2 von der Steuereinheit 50 zur Regelung des Flusses an den Lösungsbeutel 11 verwendet. Eine bilanzierende Pumpe und ein Flusssensor sind nicht vorgesehen.

In der dritten Ausführungsform gemäß Figur 6b ist die Steuereinheit 50 ausgebildet, die in den Lösungsbeutel 11 gefüllte Flüssigkeitsmenge anhand der bilanzierenden Pumpen 9a-9c zusätzlich zu den Klemmen 5a-5c zu steuern und das Signal der Waage 2 lediglich zur Überprüfung der in den Lösungsbeutel 11 gefüllte Flüssigkeitsmenge heranzuziehen.

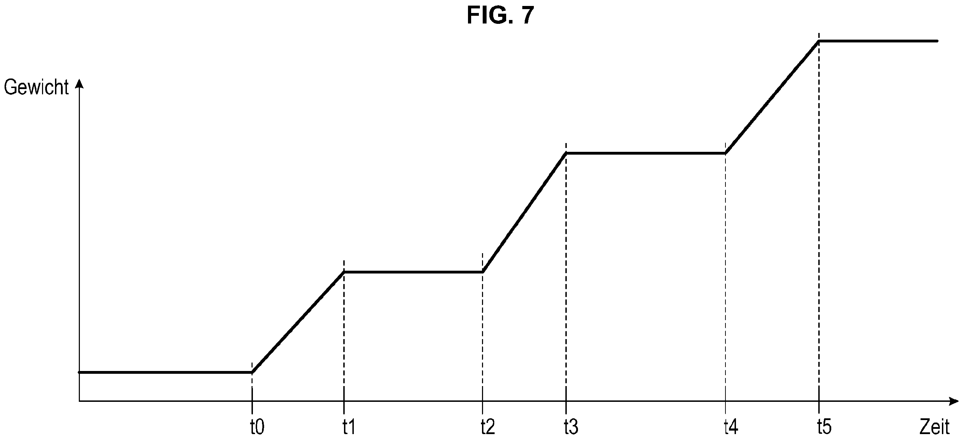

Figur 7 zeigt ein Verlaufsdiagramm für das Beutelgewicht in der zweiten und der dritten Ausführungsform der Erfindung. In beiden diesen Ausführungsformen ist die Steuereinheit ausgebildet, eine sequentielle Befüllung des Lösungsbeutels 11 mit den unterschiedlichen Lösungsbestandteilen zu veranlassen, nämlich entweder gravimetrisch anhand der Klemmen 5a-5c (zweite Ausführungsform gemäß Figur 6a) oder aktiv anhand der Pumpen 9a-9c und Klemmen 5a-5c (dritte Ausführungsform gemäß Figur 6b). Zum Zeitpunkt t0 wird ein Befüllschritt mit ein Konzentrat enthaltend ein Osmotikum (hier: Glukose) aus Reservoir 4a gestartet, der im Zeitpunkt t1 endet. Zum Zeitpunkt t2 wird ein Befüllschritt mit einem Elektrolyt- und Bufferkonzentrat aus Reservoir 4b gestartet, der im Zeitpunkt t3 endet. Zum Zeitpunkt t4 wird ein Befüllschritt mit Wasser aus Reservoir 4c gestartet, der im Zeitpunkt t5 endet.

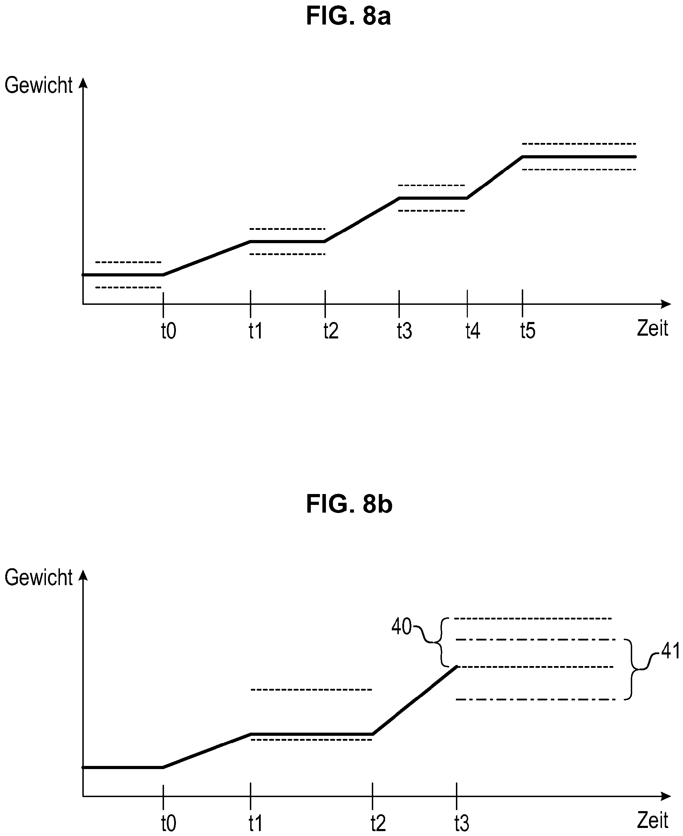

In der dritten Ausführungsform gemäß Figur 6b ist vorgesehen, dass die Steuereinheit 50 anhand der Waage 2 das Gewicht des Lösungsbeutels vor dem Befüllvorgang und nach jedem Schritt des sequentiellen des Befüllvorgangs dahingehend verifiziert, ob eine korrekte Flüssigkeitsmenge in den Lösungsbeutel 11 geflossen ist. Auch hier sind für jede Messung Sollbereiche vorgesehen, wie dies in Figur 8a veranschaulicht ist. Sollte das Gewicht des Beutels 11 außerhalb des Sollbereichs liegen, wird er von der Steuereinheit 50 nicht für den nächsten Schritt freigegeben, in einer Weise, wie dies bereits oben in anderem Zusammenhang beschrieben wurde.

Die Grenzen der jeweiligen Sollbereiche sind in dieser Ausführungsform nicht starr definiert, sondern werden durch Addition festgelegter Beträge zu dem im vorhergehenden Schritt ermittelten, tatsächlichen Gewicht des Beutels 11 individuell bestimmt. So kann vermieden werden, dass die Gewichtsverifikation um einen Betrag einer früheren Abweichung vom Sollwert verfälscht wird. Dies ist in Figur 8b veranschaulicht. Dort ist ein Szenario wiedergegeben, in dem das Beutelgewicht nach Zeitpunkt t1 innerhalb des Sollbereichs liegt, jedoch nicht genau am Sollwert sondern nahe dem unteren Ende des Sollbereichs. Das Bezugszeichen 40 bezeichnet nun den unkorrigierten Sollbereich für das Beutelgewicht nach Zeitpunkt t3, der unter der hier nicht erfüllten Voraussetzung zutreffen würde, dass das Beutelgewicht im vorhergehenden Schritt, d.h., nach dem Zeitpunkt t1, genau dem Sollwert entsprechen würde. Das Bezugszeichen 41 bezeichnet den korrigierten Sollbereich, der sich an einem korrigierten Sollwert für das Beutelgewicht nach Zeitpunkt t3 orientiert, das durch Addition einer Solldifferenz zum tatsächlichen Beutelgewicht im vorangehenden Schritt, also zwischen Zeitpunkten t1 und t2, erhalten wird.

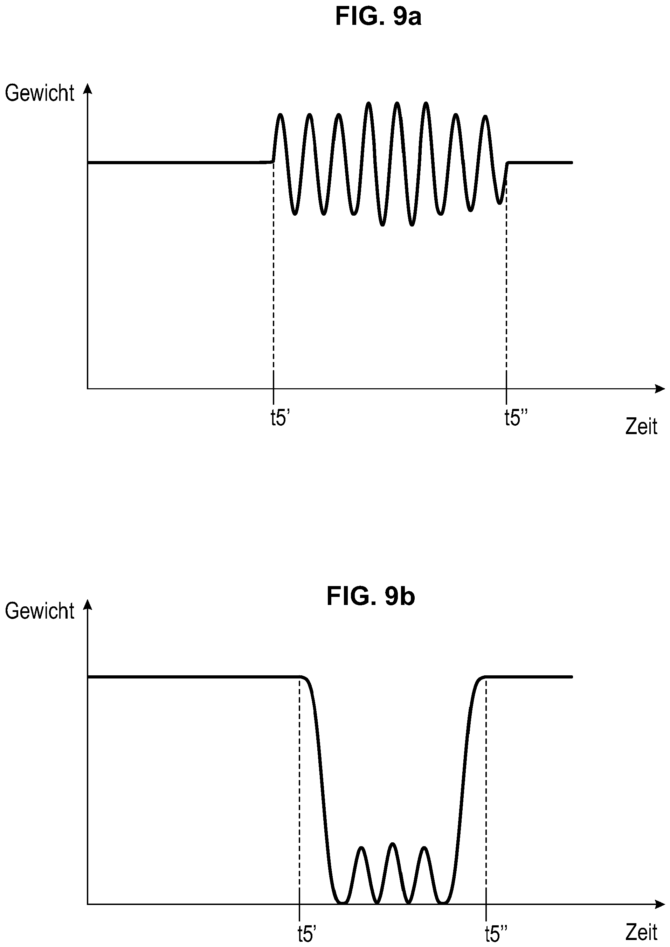

Figuren 9a und 9b zeigen vergrößerte Ansichten des Verlaufsdiagramms für die zweite Ausführungsform nach Zeitpunkt t5, also nachdem der Lösungsbeutel 11 mit allen drei Teillösungen befüllt wurde und der Befüllvorgang beendet ist. Es ist erkennbar, dass in beiden Fällen eine Signaländerung zwischen Zeitpunkten t5' und t5" eintritt. Diese Störung ist repräsentativ für ein Schütteln des in der Waagschale 3 liegenden Beutels 11 (Figur 9a) bzw. für eine kurzzeitige Entnahme des gefüllten Beutels 11 aus der Waagschale 3 (Figur 9b).

Diese Störungen resultieren daraus, dass in den jeweiligen Ausführungsformen die Steuereinheit ausgebildet ist, nach dem Ende des Befüllvorgangs ein Signal auszugeben, um eine Bewegung des befüllten Lösungsbeutels 11 zu fordern. So soll der Anwender veranlasst werden, den Lösungsbeutel 11 nach dessen vollständiger Befüllung zur homogenen Durchmischung der Lösungsbestandteile zu bewegen. Die Steuereinheit 50 kann dann anhand der Waage 2 verifizieren, ob eine solche Bewegung tatsächlich vorgenommen wurde, nämlich anhand einer Erkennung der in Figuren 9a bzw. 9b gezeigten Störungsbilder. Sollte nach Aufforderung kein solches Störungsbild erkannt werden, wird der befüllte Beutel 11 nicht für die Therapie freigegeben.

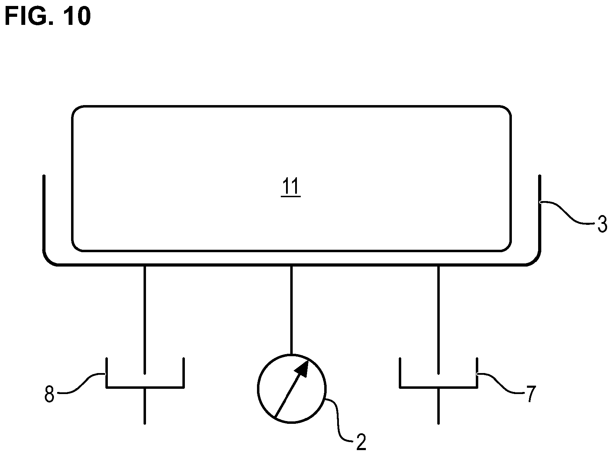

Figur 10 zeigt eine modifizierte Variante mit zwei Aktoren 7 und 8 zum Schütteln der Waagschale 3. In dieser Ausführungsform können die Waagschale 3 und mithin der darin angeordnete Lösungsbeutel 11 vom Gerät nach Beendigung des Füllvorgangs selbsttätig geschüttelt werden, ohne dass der Benutzer dazu aufgefordert werden muss bzw. dies erledigen muss.

## Patentansprüche

1. Gravimetrische Vorrichtung (1) zur Herstellung einer Peritonealdialyselösung am Anwendungsort umfassend ein Disposable (10) mit einem Lösungsbeutel (11) und damit verbundenen Füll- und Patientenleitungen (12,13), eine mit der Füllleitung (12) verbundene Bereitstellungseinheit (4) für Flüssigkeit, eine Steuereinheit (50) und Aktoren zur Regelung eines Flusses von der Bereitstellungseinheit (4) zum Disposable (10),
**dadurch gekennzeichnet,**
**dass** die Vorrichtung (1) ferner eine Waage (2) umfasst, an welcher der Lösungsbeutel (11) des Disposables (10) angeordnet ist, wobei die Steuereinheit (50) ausgebildet ist, anhand der Aktoren eine Befüllung des Lösungsbeutels (11) mit Flüssigkeit von der Bereitstellungseinheit (4) zu veranlassen und anhand der Waage (2) die Menge der in den Lösungsbeutel (11) geflossenen Flüssigkeit zu bestimmen und dazu ausgebildet ist, den Befüllvorgang nach Erreichen einer vorgegebenen Füllmenge zu stoppen und anhand der Waage (2) zu verifizieren, ob das Gewicht des Beutels (11) nach dem Stopp des Befüllvorgangs in einem Sollbereich liegt, und weiter dazu ausgebildet ist, die Grenzen des Sollbereichs durch Addition eines bestimmten Betrags zum ermittelten Ausgangsgewicht des Beutels (11) oder zu einem nach dem vorhergehenden Schritt ermittelten Gewicht des Beutels (11) festzulegen.

2. Vorrichtung (1) nach Anspruch 1, **dadurch gekennzeichnet, dass** die Vorrichtung eine Pumpe (9) umfasst und die Steuereinheit (50) ausgebildet ist, eine aktive Förderung der Flüssigkeit an den Lösungsbeutel (11) zu veranlassen.

3. Vorrichtung (1) nach Anspruch 1, **dadurch gekennzeichnet, dass** die Vorrichtung einen Flussregler umfasst und die Steuereinheit (50) ausgebildet ist, anhand des Flussreglers einen gravimetrischen Fluss der Flüssigkeit in den Lösungsbeutel (11) zu veranlassen.

4. Vorrichtung (1) nach einem der vorhergehenden Ansprüche, **dadurch gekennzeichnet, dass** die Steuereinheit (50) ausgebildet ist, die in den Lösungsbeutel (11) gefüllte Flüssigkeitsmenge anhand einer bilanzierenden Pumpe zu steuern oder auf der Grundlage des Signals eines Flusssensors zu regeln und das Signal der Waage (2) lediglich zur Überprüfung der in den Lösungsbeutel (11) gefüllte Flüssigkeitsmenge heranzuziehen.

5. Vorrichtung (1) nach einem der Ansprüche 1 bis 3, **dadurch gekennzeichnet, dass** die Steuereinheit (50) ausgebildet ist, die in den Lösungsbeutel (119 gefüllte Flüssigkeitsmenge anhand des Signals der Waage (2) zu regeln.

6. Vorrichtung (1) nach einem der vorhergehenden Ansprüche, **dadurch gekennzeichnet, dass** der Lösungsbeutel (11) mit einem Dialyseflüssigkeits-Konzentrat vorgefüllt ist, dass es sich bei der Bereitstellungseinheit (4) um eine Wasseraufbereitungseinheit handelt, und dass es sich bei der in den Lösungsbeutel (11) gefüllten Flüssigkeit um Wasser handelt.

7. Vorrichtung (1) nach einem der Ansprüche 1 bis 5, **dadurch gekennzeichnet, dass** die Bereitstellungseinheit (4) ausgebildet ist, wenigstens zwei unterschiedliche fluide Lösungsbestandteile bereitzustellen, und die Steuereinheit (50) ausgebildet ist, eine sequentielle Befüllung des Lösungsbeutels (11) mit den unterschiedlichen Lösungsbestandteilen zu veranlassen.

8. Vorrichtung (1) nach einem der vorhergehenden Ansprüche, **dadurch gekennzeichnet, dass** die Steuereinheit (50) ausgebildet ist, ein Signal auszugeben und/oder einen Start des Befüllvorgangs zu unterbinden, wenn das anhand der Waage (2) ermittelte Ausgangsgewicht des Beutels einen Schwellenwert unter- oder überschreitet.

9. Vorrichtung (1) nach einem der vorhergehenden Ansprüche, **dadurch gekennzeichnet, dass** die Steuereinheit (50) ausgebildet ist, nach dem Ende des Befüllvorgangs ein Signal auszugeben, um eine Bewegung des befüllten Lösungsbeutels (11) zu fordern, und anhand der Waage (2) zu verifizieren, ob der Lösungsbeutel (11) tatsächlich bewegt wurde.

10. Vorrichtung (1) nach einem der vorhergehenden Ansprüche, **dadurch gekennzeichnet, dass** die Vorrichtung (1) einen Aktor aufweist, der ausgebildet ist, um den eingelegten Lösungsbeutel (11) bewegen zu können, und dass die Steuereinheit (50) ausgebildet ist, den Lösungsbeutel (11) nach dem Ende des Befüllvorgangs zu bewegen.

11. Vorrichtung (1) nach einem der vorhergehenden Ansprüche, **dadurch gekennzeichnet, dass** die Steuereinheit (50) ausgebildet ist, ein Signal auszugeben und/oder die Pumpe (9) der Bereitstellungseinheit (4) zu stoppen und/oder die Füllklemme zu schließen, wenn während des Befüllvorgangs anhand der Waage (2) eine Stagnation der Gewichtszunahme oder eine Gewichtsabnahme des Lösungsbeutels (11) erkannt wird.

12. Vorrichtung (1) nach einem der vorhergehenden Ansprüche, **dadurch gekennzeichnet, dass** die Vorrichtung (1) eine Heizung aufweist und die Steuereinheit (50) ausgebildet ist, den Beutel (11) während des Befüllvorgangs oder nach dem Befüllvorgang zu erwärmen.

13. Vorrichtung (1) nach einem der vorhergehenden Ansprüche, **dadurch gekennzeichnet, dass** in der Füllleitung (12) ein Rückschlagventil angeordnet ist, das einen Fluss vom Beutel (11) zur Wasseraufbereitungseinheit (4) unterbindet.

14. Vorrichtung (1) nach einem der vorhergehenden Ansprüche, **dadurch gekennzeichnet, dass** es sich bei der Vorrichtung (1) um ein Dialysegerät handelt.

15. Vorrichtung (1) nach Anspruch 14, **dadurch gekennzeichnet, dass** die Vorrichtung eine Füllklemme und eine Patientenklemme aufweist, wobei die Füllleitung (12) des Disposables (10) in die Fülllklemme und die Patientenleitung (13) in die Patientenklemme eingelegt ist, und wobei die Steuereinheit (50) ausgebildet ist, die Befüllung des Lösungsbeutels (11) bei offener Füllklemme und geschlossener Patientenklemme vorzunehmen.

16. Vorrichtung (1) nach Anspruch 14 oder 15, **dadurch gekennzeichnet, dass** die Vorrichtung ein weiteres Disposable (10) oder das Disposable (10) eine zusätzliche Kavität für aus dem Patienten abfließendes Dialysat aufweist, das oder die auf derselben Waage (2) angeordnet ist wie der Lösungsbeutel (11), wobei die Steuereinheit (50) ausgebildet ist, die Menge des im Rahmen einer Dialysebehandlung von dem Patienten abfließenden Dialysats gravimetrisch zu bestimmen.

17. Vorrichtung (1) nnach einem der vorhergehenden Ansprüche, **dadurch gekennzeichnet, dass** die Vorrichtung ein graphisches Benutzerinterface mit vorzugsweise einem Touchscreen aufweist und die Steuereinheit (50) ausgebildet ist, am Benutzerinterface den Fortschritt des Befüllvorgangs zu visualiseren und/oder Signale auszugeben.

18. Verfahren zur Herstellung einer Peritonealdialyselösung am Anwendungsort unter Verwendung einer Vorrichtung (1) nach einem der vorhergehenden Ansprüche,
**dadurch gekennzeichnet,**
**dass** anhand der Aktoren eine Befüllung des Lösungsbeutels (11) mit Flüssigkeit von der Bereitstellungseinheit veranlasst wird und anhand der Waage (2) die Menge der in den Lösungsbeutel geflossenen Flüssigkeit bestimmt wird.

## Claims

1. A gravimetric apparatus (1) for preparing a peritoneal dialysis solution at the point of use comprising a disposable (10) having a solution bag (11) and associated filling lines and patient lines (12, 13) connected thereto; a storage unit (4) for liquid connected to the filling line (12); a control unit (50); and actuators for regulating a flow from the storage unit (4) to the disposable (10),
**characterized in that**
the apparatus (1) furthermore comprises a scale (2) at which the solution bag (11) of the disposable (10) is arranged, with the control unit (50) being configured to initiate a filling of the solution bag (11) with liquid from the storage unit (4) using the actuators and to determine the quantity of the liquid that has flowed into the solution bag (11) using the scale (2) and is configured to stop the filling procedure after reaching a predefined filling quantity and to verify by means of the scale (2) whether the weight of the bag (11) is within a desired range after the stopping of the filling procedure and is further configured to fix the limits of the desired range by addition of a specific amount to the determined starting weight of the bag (11) or to a weight of the bag (11) determined after the preceding step.

2. An apparatus (1) in accordance with claim 1, **characterized in that** the apparatus comprises a pump (9) and that the control unit (50) is configured to initiate an active conveying of the liquid to the solution bag (11).

3. An apparatus (1) in accordance with claim 1, **characterized in that** the apparatus comprises a flow regulator and that the control unit (50) is configured to initiate a gravimetric flow of the liquid into the solution bag (11) using the flow regulator.

4. An apparatus (1) in accordance with one of the preceding claims, **characterized in that** the control unit (50) is configured to control the liquid quantity filled into the solution bag (11) using a balancing pump or to regulate it on the basis of the signal of a flow sensor and only to make use of the signal of the scale (2) to check the quantity of liquid filled into the solution bag (11).

5. An apparatus (1) in accordance with one of the claims 1 to 3, **characterized in that** the control unit (50) is configured to regulate the liquid quantity filled into the solution bag (11) using the signal of the scale (2).

6. An apparatus (1) in accordance with one of the preceding claims, **characterized in that** the solution bag (11) is pre-filled with a dialysis fluid concentrate; **in that** the storage unit (4) is a water preparation unit; and **in that** the liquid filled into the solution bag (11) is water.

7. An apparatus (1) in accordance with one of the claims 1 to 5, **characterized in that** the storage unit (4) is configured to provide at least two different fluid solution components; and **in that** the control unit (50) is configured to initiate a sequential filling of the solution bag (11) with the different solution components.

8. An apparatus (1) in accordance with one of the preceding claims, **characterized in that** the control unit (50) is configured to output a signal and/or to suppress a start of the filling procedure when the starting weight of the bag determined using the scale (2) falls below or exceeds a threshold value.

9. An apparatus (1) in accordance with one of the preceding claims, **characterized in that** the control unit (50) is configured to output a signal after the end of the filling procedure to request a movement of the filled solution bag (11) and to verify by means of the scale (2) whether the solution bag (11) has actually been moved.

10. An apparatus (1) in accordance with one of the preceding claims, **characterized in that** the apparatus (1) has an actuator that is configured to be able to move the inserted solution bag (11) and that the control unit (50) is configured to move the solution bag (11) after the end of the filling procedure.

11. An apparatus (1) in accordance with one of the preceding claims, **characterized in that** the control unit (50) is configured to output a signal and/or to stop the pump (9) of the storage unit (4) and/or to close the filling clamp if a stagnation of the weight increase or a weight reduction of the solution bag (11) is recognized with reference to the scale (2) during the filling procedure.

12. An apparatus (1) in accordance with one of the preceding claims, **characterized in that** the apparatus (1) has a heating and the control unit (50) is configured to heat the bag (11) during the filling procedure or after the filling procedure.

13. An apparatus (1) in accordance with one of the preceding claims, **characterized in that** a check valve is arranged in the filling line (12) and suppresses a flow from the bag (11) to the water preparation unit (4).

14. An apparatus (1) in accordance with one of the preceding claims, **characterized in that** the apparatus (1) is a dialysis machine.

15. An apparatus (1) in accordance with claim 14, **characterized in that** the apparatus has a filling clamp and a patient clamp, wherein the filling line (12) of the disposable (10) is inserted into the filling clamp and the patient line (13) is inserted into the patient clamp, and wherein the control unit (50) is configured to carry out the filling of the solution bag (11) with an open filling clamp and a closed patient clamp.

16. An apparatus (1) in accordance with claim 14 or claim 15, **characterized in that** the apparatus has a further disposable (10) or that the disposable (10) has an additional cavity for dialyzate flowing out of the patient, said disposable (10) or cavity being arranged on the same scale (2) as the solution bag (11), with the control unit (50) being configured to gravimetrically determine the quantity of the dialyzate flowing off from the patient as part of a dialysis treatment.

17. An apparatus (1) in accordance with one of the preceding claims, **characterized in that** the apparatus has a graphical user interface preferably having a touch screen and that the control unit (50) is configured to visualize the progression of the filling procedure at the user interface and/or to output signals.

18. A method of preparing a peritoneal dialysis solution at the point of use using an apparatus (1) in accordance with one of the preceding claims,
**characterized in that**
a filling of the solution bag (11) with liquid is initiated by the storage unit using the actuators and the quantity of the liquid that has flowed into the solution bag is determined using the scale (2).

## Revendications

1. Dispositif gravimétrique (1) destiné à la fabrication d'une solution de dialyse péritonéale sur le lieu d'utilisation, comprenant un article à usage unique (10) avec une poche de solution (11) et des conduites de remplissage et de liaison au patient (12, 13) reliées à celle-ci, une unité de préparation (4) pour liquide reliée à la conduite de remplissage (12), une unité de commande (50) et des actionneurs pour la régulation d'un flux depuis l'unité de préparation (4) jusqu'à l'article à usage unique (10),
**caractérisé en ce que**
le dispositif (1) comprend en outre une balance (2), sur laquelle est disposée la poche de solution (11) de l'article à usage unique (10), l'unité de commande (50) étant conçue pour, au moyen des actionneurs, déclencher un remplissage de la poche de solution (11) avec du liquide provenant de l'unité de préparation (4) et, au moyen de la balance (2), déterminer la quantité de liquide qui s'est écoulé dans la poche de solution (11) et étant conçue pour arrêter le processus de remplissage lorsqu'une quantité de remplissage prédéfinie a été atteinte et pour vérifier au moyen de la balance (2) si le poids de la poche (11) se trouve dans une plage de consigne après l'arrêt du processus de remplissage, et étant en outre conçue pour définir les limites de la plage de consigne en ajoutant une grandeur donnée au poids initial déterminé de la poche (11) ou à un poids de la poche (11) déterminé après l'étape précédente.

2. Dispositif (1) selon la revendication 1, **caractérisé en ce que** le dispositif comprend une pompe (9) et l'unité de commande (50) est conçue pour déclencher un transport actif du liquide jusqu'à la poche de solution (11).

3. Dispositif (1) selon la revendication 1, **caractérisé en ce que** le dispositif comprend un régulateur de flux et l'unité de commande (50) est conçue pour déclencher un flux gravimétrique du liquide jusqu'à la poche de solution (11) au moyen du régulateur de débit.

4. Dispositif (1) selon l'une des revendications précédentes, **caractérisé en ce que** l'unité de commande (50) est conçue pour commander la quantité de liquide remplie dans la poche de solution (11) au moyen d'une pompe d'équilibrage ou la réguler en fonction d'un signal d'un capteur de flux et tenir compte du signal de la balance (2) seulement pour vérifier la quantité de liquide remplie dans la poche de solution (11).

5. Dispositif (1) selon l'une des revendications 1 à 3, **caractérisé en ce que** l'unité de commande (50) est conçue pour réguler la quantité de liquide remplie dans la poche de solution (11) au moyen du signal de la balance (2).

6. Dispositif (1) selon l'une des revendications précédentes, **caractérisé en ce que** la poche de solution (11) est préremplie avec un concentré de liquide de dialyse, **en ce que** l'unité de préparation (4) est une unité de traitement d'eau et **en ce que** le liquide rempli dans la poche de solution (11) est de l'eau.

7. Dispositif (1) selon l'une des revendications 1 à 5, **caractérisé en ce que** l'unité de préparation (4) est conçue pour la préparation d'au moins deux composants de solution fluides différents, et l'unité de commande (50) est conçue pour déclencher un remplissage séquentiel de la poche de solution (11) avec les différents composants de solution.

8. Dispositif (1) selon l'une des revendications précédentes, **caractérisé en ce que** l'unité de commande (50) est conçue pour émettre un signal et/ou empêcher un démarrage du processus de remplissage quand le poids initial de la poche déterminé au moyen de la balance (2) est inférieur ou supérieur à une valeur seuil.

9. Dispositif (1) selon l'une des revendications précédentes, **caractérisé en ce que** l'unité de commande (50) est conçue pour émettre un signal après la fin du processus de remplissage pour provoquer un déplacement de la poche de solution (11) remplie et pour vérifier au moyen de la balance (2) si la poche de solution (11) a effectivement été déplacée.

10. Dispositif (1) selon l'une des revendications précédentes, **caractérisé en ce que** le dispositif (1) présente un actionneur qui est conçu pour pouvoir déplacer la poche de solution (11) introduite et **en ce que** l'unité de commande (50) est conçue pour déplacer la poche de solution (11) après la fin du processus de remplissage.

11. Dispositif (1) selon l'une des revendications précédentes, **caractérisé en ce que** l'unité de commande (50) est conçue pour émettre un signal et/ou arrêter la pompe (9) de l'unité de préparation (4) et/ou fermer la pince de remplissage quand une stagnation de l'augmentation du poids ou une diminution du poids de la poche de solution (11) est détectée au moyen de la balance (2) pendant le processus de remplissage.

12. Dispositif (1) selon l'une des revendications précédentes, **caractérisé en ce que** le dispositif (1) présente un chauffage et l'unité de commande (50) est conçue pour chauffer la poche (11) pendant le processus de remplissage ou après le processus de remplissage.

13. Dispositif (1) selon l'une des revendications précédentes, **caractérisé en ce qu'**un clapet antiretour, qui empêche un écoulement de la poche (11) à l'unité de traitement d'eau (4), est disposé dans la conduite de remplissage (12).

14. Dispositif (1) selon l'une des revendications précédentes, **caractérisé en ce que** le dispositif (1) est un appareil de dialyse.

15. Dispositif (1) selon la revendication 14, **caractérisé en ce que** le dispositif présente une pince de remplissage et une pince côté patient, la conduite de remplissage (12) de l'article à usage unique (10) étant insérée dans la pince de remplissage et la conduite de liaison au patient (13) dans la pince côté patient et l'unité de commande (50) étant conçue pour effectuer le remplissage de la poche de solution (11) lorsque la pince de remplissage est ouverte et la pince côté patient est fermée.

16. Dispositif (1) selon la revendication 14 ou 15, **caractérisé en ce que** le dispositif présente un autre article à usage unique (10) ou l'article à usage unique (10) présente une cavité supplémentaire pour du dialysat s'écoulant hors du patient, lequel ou laquelle est disposé(e) sur la même balance (2) que la poche de solution (11), l'unité de commande (50) étant conçue pour déterminer par gravimétrie la quantité du dialysat s'écoulant hors du patient dans le cadre d'un traitement de dialyse.

17. Dispositif (1) selon l'une des revendications précédentes, **caractérisé en ce que** le dispositif présente une interface utilisateur graphique avec de préférence un écran tactile et l'unité de commande (50) est conçue pour visualiser l'avancement du processus de remplissage sur l'interface utilisateur et/ou émettre des signaux.

18. Procédé de fabrication d'une solution de dialyse péritonéale sur le lieu d'utilisation au moyen d'un dispositif (1) selon l'une des revendications précédentes,
**caractérisé en ce que**
un remplissage de la poche de solution (11) avec du liquide provenant de l'unité de préparation est déclenché au moyen des actionneurs et la quantité du liquide qui s'est écoulé dans la poche de solution est déterminée au moyen de la balance (2).
